# EUROPEAN PATENT SPECIFICATION

(11) **EP 1 315 723 B1**
(45) Date of publication and mention of the grant of the patent: **27.07.2005**
(21) Application number: 01963254.6
(22) Date of filing: 05.09.2001
(51) Int. Cl.: C07D 417/12, A61K 31/44, A61P 3/00

(54) **THE HYDROCHLORIDE SALT OF 5[4-[2-(N-METHYL-N-(2-PYRIDYL)AMINO)ETHOXY]BENZYL]THIAZOLIDINE-2,4-DIONE**
DAS HYDROCHLORIDSALZ DES 5[4-[2-(N-METHYL-N-(2-PYRIDYL)AMINO)ETHOXY]BENZYL]THIAZOLIDIN-2,4-DIONS
SEL HYDROCHLORIDE DE 5- 4- 2-(N-METHYL-N-(2-PYRIDYL)AMINO)ETHOXY]BENZYL]THIAZOLIDINE-2,4-DIONE

(30) Priority: 06.09.2000 GB 0021865
(43) Date of publication of application: 04.06.2003
(73) Proprietor: SMITHKLINE BEECHAM PLC, Brentford, Middlesex TW8 9GS (GB)
(72) Inventor: CRAIG, Andrew Simon, Glaxo SmithKline, Tonbridge, Kent TN11 9AN (GB); MILLAN, Michael, Glaxo SmithKline, Tonbridge, Kent TN11 9AN (GB)
(74) Representative: Rutter, Keith
(86) International application number: PCT/GB2001/003991
(87) International publication number: WO 2002/020519

(56) References cited:
- EP-A- 0 306 228
- WO-A-94/05659

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound I").

International Patent Application, Publication Number WO94/05659 discloses certain salts of the compounds of EP 0,306,228 and in particular the maleic acid salt.

It has now been discovered that Compound I forms a novel hydrochloride salt (hereinafter also referred to as the "Hydrochloride") that is particularly stable and hence is suitable for bulk preparation and handling. The Hydrochloride also has a high melting point, shows particularly good aqueous solubility and possesses good bulk flow properties. The Hydrochloride is therefore surprisingly amenable to large scale pharmaceutical processing and especially to large scale miling.

The novel form can be prepared by an efficient, economic and reproducible process particularly suited to large-scale preparation.

The novel Hydrochloride also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione hydrochloride characterised in that it provides:
(i) an infrared spectrum with bands observed at: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525, and 505 cm⁻¹; and/or
(ii) a Raman spectrum with bands observed at: 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340, and 300 cm⁻¹; and/or
(iii) an X-Ray powder diffraction pattern (XRPD) with characteristic XRPD angles and relative intensities (%) of 5.5 (15.4), 6.7 (14.1), 8.7 (3.3), 11.1 (39.4), 12.0 (9.2), 12.7 (20.3), 13.5, (40.8), 14.7 (40.8), 15.1 (14.5), 15.5 (38.1), 16.3 (30.2), 16.8 (18.5), 17.4 (27.1), 17.7 (34.6), 18.2 (26.7), 18.5 (26), 18.8 (38.8), 19.8 (31), 20.3 (11.7), 21.2 (16.7), 21.7 (26.4), 21.9 (26), 22.5 (14.7), 23.2 (26.2), 23.6 (90.8), 24.2 (100), 25.1, (49.6), 25.5 (68.5), 25.9 (29.3), 26.4 (16.8), 27.3 (25.6), 27.8 (15.4), 28.1 (28.8), 28.8 (21.6), 29.3 (44.1), 29.9 (20.9), 30.5 (17.8), 30.9 (16.8), 31.8 (24), 32.9 (18.7), 33.1 (22), and 34.3 (22.7) °2-theta; and
(iv) a Solid State ¹³C NMR spectrum with chemical shifts observed at: 39.0, 52.5, 54.9, 64.1, 67.3, 111.6, 114.4, 131.0, 137.0, 144.2, 151.3, 156.4, 158.5, 171.6, 175.5, and 177.9 ppm.

In one favoured aspect, the Hydrochloride provides an infrared spectrum with bands observed at: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525, and 505 cm⁻¹.

In a further aspect, the Hydrochloride provides a Raman spectrum with bands observed at: 3100, 3056, 2931, 1744, 1610, 1586, 1544, 1461, 1440, 1377, 1351, 1319, 1289, 1253, 1232, 1208, 1177, 1495, 1028, 998, 980, 925, 900, 840, 825, 772, 740, 714, 664, 637, 619, 606, 469, 430, 397, 340, and 300 cm^{-1.}

In one favoured aspect, the Hydrochloride provides an X-Ray powder diffraction pattern (XRPD) with characteristic XRPD angles and relative intensities (%) of 5.5 (15.4), 6.7 (14.1), 8.7 (3.3), 11.1 (39.4), 12.0 (9.2), 12.7 (20.3), 13.5, (40.8), 14.7 (40.8), 15.1 (14.5), 15.5 (38.1), 16.3 (30.2), 16.8 (18.5), 17.4 (27.1), 17.7 (34.6), 18.2 (26.7), 18.5 (26), 18.8 (38.8), 19.8 (31), 20.3 (11.7), 21.2 (16.7), 21.7 (26.4), 21.9 (26), 22.5 (14.7), 23.2 (26.2), 23.6 (90.8), 24.2 (100), 25.1, (49.6), 25.5 (68.5), 25.9 (29.3), 26.4 (16.8), 27.3 (25.6), 27.8 (15.4), 28.1 (28.8), 28.8 (21.6), 29.3 (44.1), 29.9 (20.9), 30.5 (17.8), 30.9 (16.8), 31.8 (24), 32.9 (18.7), 33.1 (22), and 34.3 (22.7) °2-theta;.

In one favoured aspect, the Hydrochloride provides a Solid State ¹³C NMR spectrum with chemical shifts observed at: 39.0, 52.5, 54.9, 64.1, 67.3, 111.6, 114.4, 131.0, 137.0, 144.2, 151.3, 156.4, 158.5, 171.6, 175.5, and 177.9 ppm..

The Hydrochloride also provides provides a melting point in the range of from 160 to 168°C, such as 163 to 167 °C, for example 167 °C

The present invention encompasses the Hydrochloride isolated in pure form or when admixed with other materials. Thus in one aspect there is provided the Hydrochloride in isolated form.

In a further aspect there is provided the Hydrochloride in a purified form.

In yet a further aspect there is provided the Hydrochloride in crystalline form.

Also, the invention provides the Hydrochloride in a solid pharmaceutically acceptable form, such as a solid dosage form, especially when adapted for oral administration.

Moreover, the invention also provides the Hydrochloride in a pharmaceutically acceptable form, especially in bulk form, such form being particularly capable of being milled. The invention therefor also provides the the Hydrochloride in a milled form.

Furthermore, the invention provides the Hydrochloride in a pharmaceutically acceptable form, especially in bulk form, such form having good flow properties, especially good bulk flow properties.

The invention also provides a process for preparing the Hydrochloride, characterised in that 5-[4-[2-(N-methyl-N-(2-pyridyl)amino)ethoxy]benzyl]thiazolidine-2,4-dione (Compound I) or salt thereof, preferably dispersed or dissolved in a suitable solvent, is reacted with a source of chloride ion; and thereafter the Hydrochloride is recovered.

Suitably, Compound (I) is used in the reaction, especially in a protonated form.

Suitably, a salt of Compound (I) is used in the reaction..

Compound (I) is preferably present in a protonated form.

A suitable solvent is an alkanol, for example propan-2-ol, or a hydrocarbon, such as toluene, a ketone, such as acetone, an ester, such as ethyl acetate, an ether such as tetrahydrofuran, a nitrile such as acetonitrile, a halogenated hydrocarbon such as dichloromethane, an organic acid such as acetic acid, or water, or a mixture thereof.

Conveniently, the source of chloride ion is a solution of hydrogen chloride in an appropriate solvent, usually the reaction solvent, for example propan-2-ol. Alternatively, the source of chloride ion may be an aqueous solution of hydrogen chloride, such as concentrated hydrochloric acid.

An alternative source of chloride ion for a salt of Compound (I) is provided by a base salt of hydrochloric acid for example ammonium chloride, or the hydrochloric acid salt of an amine, for example ethylamine or diethylamine.

The concentration of Compound (I) is preferably in the range of from 3 to 25% weight/volume, more preferably in the range of from 5 to 20%. The concentration of hydrochloric acid solutions are preferably in the range of from 3 to 50% weight/volume.

The reaction is usually carried out at ambient temperature or at an elevated temperature, although any convenient temperature that provides the required product may be employed. A preferred temperature is in the range of from 20-120°C, such as 40°C to 90°C, for example 70°C.

Recovery of the required compound generally comprises crystallisation from an appropriate solvent, conveniently the reaction solvent, usually by cooling to a temperature in the range of from 0°C to 40°C, for example 20°C. For example the Hydrochloride may be crystallised from an alcohol such as propan-2-ol, a nitrile such as acetonitrile or an ether such as tetrahydrofuran.

In one preferred form the recovery comprises initial crystallisation at an elevated temperature such as 50°C to 90°C preferably 65°C to 75°C and thereafter cooling to a second temperature, suitably in the range of 0°C to 40°C, to complete crystallisation.

Crystallisation can also be initiated by seeding with crystals of the Hydrochloride but this is not essential.

Compound (I) is prepared according to known procedures, such as those disclosed in EP 0,306,228 and WO94/05659.

When used herein the term "Tₒₙₛₑₜ" is generally determined by Differential Scanning Calorimetry and has a meaning generally understood in the art, as for example expressed in Pharmaceutical Thermal Analysis, Techniques and Applications", Ford and Timmins, 1989 as "The temperature corresponding to the intersection of the pre-transition baseline with the extrapolated leading edge of the transition".

When used herein in respect of certain compounds the term "good flow properties" is suitably characterised by the said compound having a Hausner ratio of less than or equal to 1.5, especially of less than or equal to 1.25.

"Hausner ratio" is an art accepted term.When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis,certain eating disorders, in particular the regulation of appetite and food intake in subjects suffering from disorders associated with under-eating, such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides the Hydrochloride for use as an active therapeutic substance.

More particularly, the present invention provides the Hydrochloride for use in the treatment and/or prophylaxis of diabetes mellitus, conditions associated with diabetes mellitus and certain complications thereof.

Hydrochloride may be administered per se or, preferably, as a pharmaceutical composition also comprising a pharmaceutically acceptable carrier. The formulation of the Hydrochloride is generally as disclosed for Compound (I) in the above mentioned publications.

Accordingly, the present invention also provides a pharmaceutical composition comprising the Hydrochloride and a pharmaceutically acceptable carrier therefor.

The Hydrochloride is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Hydrochloride to a human or non-human mammal in need thereof.

The compositions are formulated according to conventional methods, such as those disclosed in standard reference texts, for example the British and US Pharmacopoeias, Remington's Pharmaceutical Sciences (Mack Publishing Co.), Martindale The Complete Drug Reference (London, The Pharmaceutical Press) and Harry's Cosmeticology (Leonard Hill Books).Conveniently, the active ingredient may be administered as a pharmaceutical composition hereinbefore defined, and this forms a particular aspect of the present invention.

In a further aspect the present invention provides the use of Hydrochloride for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof the Hydrochloride may be taken in amounts so as to provide Compound I in suitable doses, such as those disclosed in EP 0,306,228 and WO94/05659.

No adverse toxicological effects are indicated in the above mentioned treatments for the compounds of the invention.

The following examples illustrate the invention but do not limit it in any way.

### Example 1 5-[4-[2-(N-methyl-N-(2-pyridyl)amino)ethoxy]benzyl]thiazolidine-2,4-dione hydrochloride

A mixture of 5-[4-[2-(N-methyl-N-(2-pyridyl)amino)ethoxy]benzyl]thiazolidine-2,4-dione (9.0 g) and propan-2-ol (180 ml) was stirred and heated to 75°C. A solution of hydrogen chloride in propan-2-ol (7.5 ml, 5-6N) was added and the mixture was stirred for 1 hour at 75°C and then cooled to 21°C. The product was collected by filtration, washed with propan-2-ol (25 ml) and dried under vacuum over phosphorus pentoxide for 16 hours to give the title compound (9.8 g) as a white crystalline solid.
Ionic chlorine content: 8.7% wt/wt
Water content (Karl-Fisher) : 0.3 % wt/wt
¹H-NMR (d⁶-DMSO): consistent with 5-[4-[2-(N-methyl-N-(2-pyridyl)amino)ethoxy]benzyl]thiazolidine-2,4-dione hydrochloride containing propan-2-ol 2.1 % wt/wt.

### Example 2 5-[4-[2-(N-methyl-N-(2-pyridyl)amino)ethoxy]benzyl]thiazolidine-2,4-dione hydrochloride

A mixture of 5-[4-[2-(N-methyl-N-(2-pyridyl)amino)ethoxy]benzyl]thiazolidine-2,4-dione (6.0 g) and propan-2-ol (120 ml) was heated to 50°C before a solution of hydrogen chloride in propan-2-ol (5.0 ml, 5-6 N) was added. The reaction temperature was raised to 70°C and stirring continued at this temperature for 2 hours, during which time crystallisation was observed to have started. After cooling to 21°C the product was collected by filtration, washed with propan-2-ol (20 ml) and dried under vacuum, over phosphorus pentoxide, to give 5-[4-[2-(N-methyl-N-(2-pyridyl)amino)ethoxy]benzyl]thiazolidine-2,4-dione hydrochloride (6.31 g) as a white crystalline solid.

### Example 3 5-[4-[2-(N-methyl-N-(2-pyridyl)amino)ethoxy]benzyl]thiazolidine-2,4-dione hydrochloride

A mixture of 5-[4-[2-(N-methyl-N-(2-pyridyl)amino)ethoxy]benzyl]thiazolidine-2,4-dione (6.0 g) and acetonitrile (120 ml) was stirred and heated to 50°C and a solution of hydrogen chloride in propan-2-ol (5.0 ml, 5-6N) was added. The reaction mixture was warmed to 70°C and stirred at this temperature for 60 minutes, during which time crystallisation was observed to have started. After cooling to 21 °C the product was collected by filtration, washed with acetonitrile (20 ml) and then dried under vacuum over phosphorus pentoxide to give 5-[4-[2-(N-methyl-N-(2-pyridyl)amino)ethoxy]benzyl]thiazolidine-2,4-dione hydrochloride (5.7 g) as a white crystalline solid.

### Example 4 5-[4-[2-(N-methyl-N-(2-pyridyl)amino)ethoxy]benzyl]thiazolidine-2,4-dione hydrochloride

A mixture of 5-[4-[2-(N-methyl-N-(2-pyridyl)amino)ethoxy]benzyl]thiazolidine-2,4-dione (6.0 g) and tetrahydrofuran (120 ml) was stirred and heated to 65°C. A solution of hydrogen chloride in propan-2-ol (5.0 ml, 5-6N) was added and the reaction mixture stirred at 65°C for 2 hours and then cooled to 21°C. The product was collected by filtration, washed with tetrahydrofuran (20 ml) and dried under vacuum over phosphorus pentoxide to give 5-[4-[2-(N-methyl-N-(2-pyridyl)amino)ethoxy]benzyl]thiazolidine-2,4-dione hydrochloride (6.25 g) as white crystalline solid.

### Example 5 5-[4-[2-(N-methyl-N-(2-pyridyl)amino)ethoxy]benzyl]thiazolidine-2,4-dione hydrochloride

Concentrated hydrochloric acid (0.85ml) was added to a stirred solution of 5-[4-[2-(N-methyl-N-(2-pyridyl)amino)ethoxy]benzyl]thiazolidine-2,4-dione (3.0 g) in propan-2-ol (90 ml) at reflux. The solution was maintained at reflux for 15 minutes and then cooled to 0°C where the precipitation of an oil was observed. The mixture was then warmed to 55°C to produce a clear solution before being cooled to 50°C to initiate crystallisation. The mixture was further cooled to 21°C and the solid was collected by filtration to afford 5-[4-[2-(N-methyl-N-(2-pyridyl)amino)ethoxy]benzyl]thiazolidine-2,4-dione hydrochloride (2.61 g) as white crystalline solid.

### Characterising data recorded for the product of Example 1.

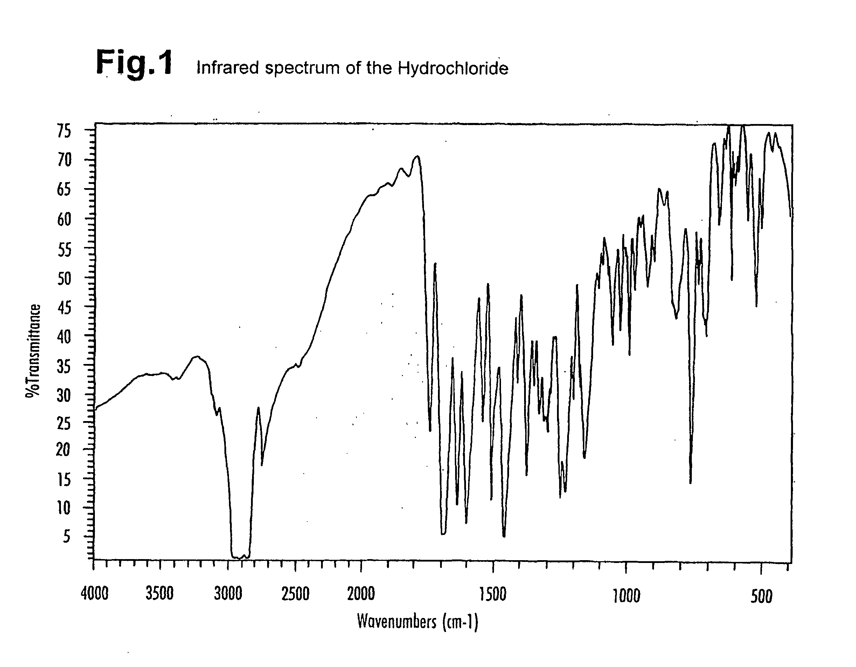

The infrared absorption spectrum of a mineral oil dispersion of the product was obtained using a Nicolet 710 FT-IR spectrometer at 2 cm⁻¹ resolution (Figure 1). Data were digitised at 1 cm⁻¹ intervals. Bands were observed at: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525, 505 cm⁻¹.

The infrared spectrum of the solid product was recorded using a Perkin-Elmer Spectrum One FT-IR spectrometer fitted with a universal ATR accessory. Bands were observed at: 2748, 1742, 1687, 1639, 1603, 1543, 1510, 1461, 1413, 1328, 1301, 1250, 1231, 1205, 1179, 1156, 1056, 1028, 996, 973, 925, 901, 817, 762, 738, 709, 661 cm⁻¹.

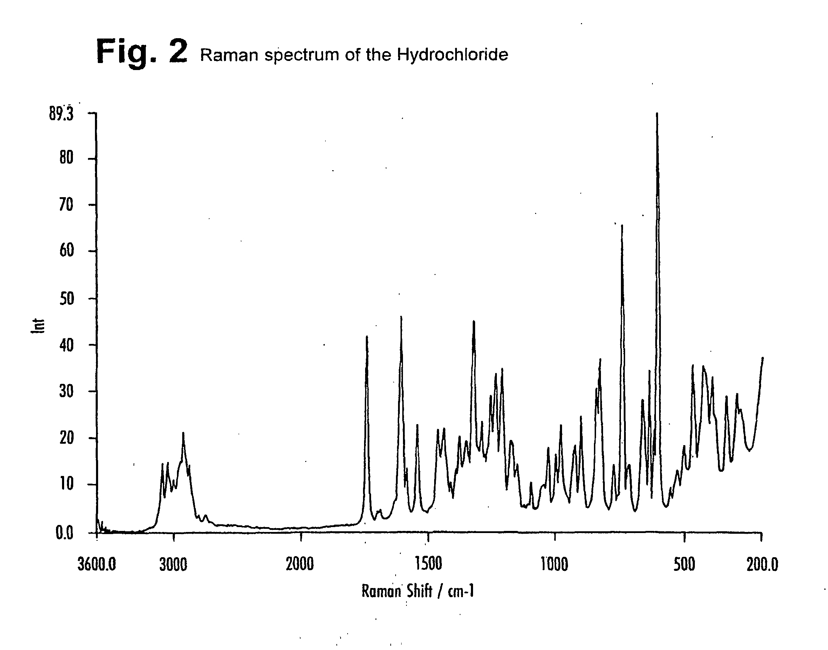

The Raman spectrum of the product (Figure 2) was recorded with the sample in a glass vial using a Perkin-Elmer 2000R FT-Raman spectrometer, at 4 cm⁻¹ resolution with excitation was from a Nd:YAG laser (1064 nm) with a power output of 400 mW. Bands were observed at: 3100,3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714,664,637,619, 606, 469, 430, 397, 340, 300 cm⁻¹.

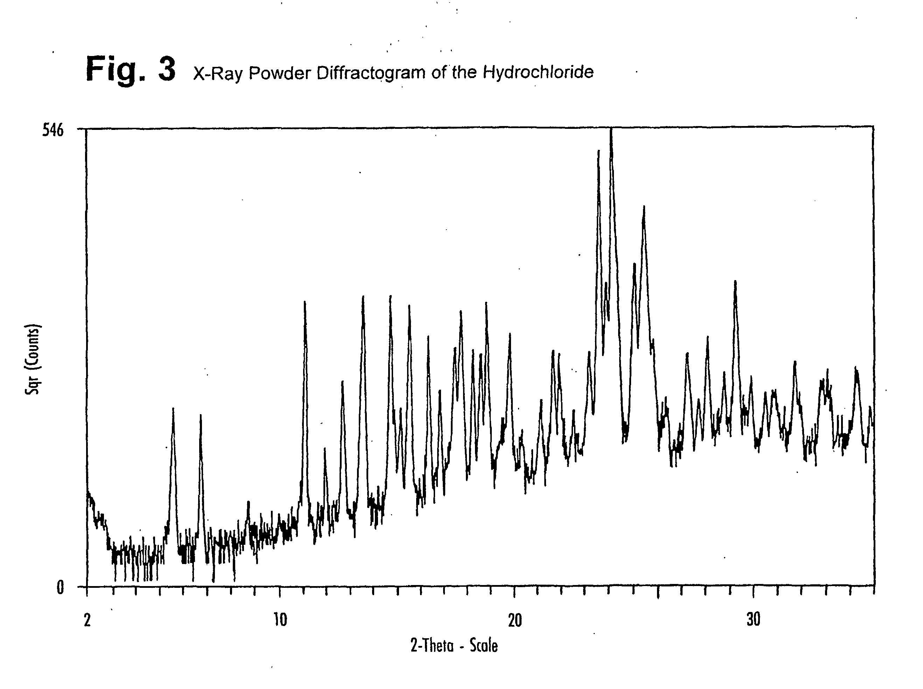

The X-Ray Powder Diffractogram pattern of the product (Figure 3) was recorded using the following acquisition conditions: Tube anode: Cu, Generator tension: 40 kV, Generator current: 40 mA, Start angle: 2.0 °2θ, End angle: 35.0 °2θ, Step size: 0.02 °2θ, Time per step: 2.5 seconds. Characteristic XRPD angles and relative intensities are recorded in Table 1.

**Table 1.**

| **Angle** | **Rel. Intensity** |
|---|---|
| **2-Theta °** | **%** |
| 5.5 | 15.4 |
| 6.7 | 14.1 |
| 8.7 | 3.3 |
| 11.1 | 39.4 |
| 12.0 | 9.2 |
| 12.7 | 20.3 |
| 13.5 | 40.8 |
| 14.7 | 40.8 |
| 15.1 | 14.5 |
| 15.5 | 38.1 |
| 16.3 | 30.2 |
| 16.8 | 18.5 |
| 17.4 | 27.1 |
| 17.7 | 34.6 |
| 18.2 | 26.7 |
| 18.5 | 26 |
| 18.8 | 38.8 |
| 19.8 | 31 |
| 20.3 | 11.7 |
| 21.2 | 16.7 |
| 21.7 | 26.4 |
| 21.9 | 26 |
| 22.5 | 14.7 |
| 23.2 | 26.2 |
| 23.6 | 90.8 |
| 24.2 | 100 |
| 25.1 | 49.6 |
| 25.5 | 68.5 |
| 25.9 | 29.3 |
| 26.4 | 16.8 |
| 27.3 | 25.6 |
| 27.8 | 15.4 |
| 28.1 | 28.8 |
| 28.8 | 21.6 |
| 29.3 | 44.1 |
| 29.9 | 20.9 |
| 30.5 | 17.8 |
| 30.9 | 16.8 |
| 31.8 | 24 |
| 32.9 | 18.7 |
| 33.1 | 22 |
| 34.3 | 22.7 |

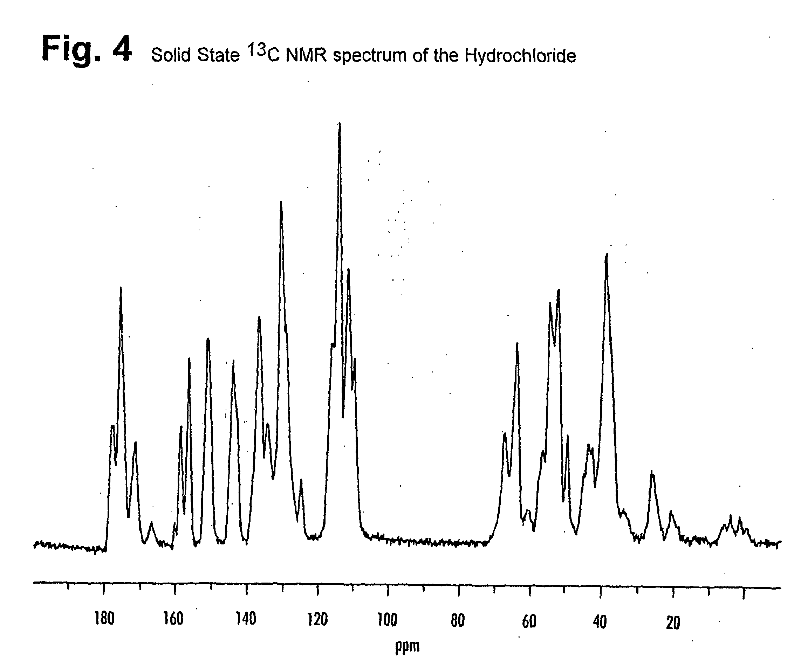

The solid-state NMR spectrum of the product (Figure 4) was recorded on a Bruker AMX36 instrument operating at 90.55 MHz: The solid was packed into a 4 mm zirconia MAS rotor fitted with a Kel-F cap and rotor spun at ca.10 kHz. The ¹³C MAS spectrum was acquired by cross-polarisation from Hartmann-Hahn matched protons (CP contact time 3 ms, repetition time 15 s) and protons were decoupled during acquisition using a two-pulse phase modulated (TPPM) composite sequence. Chemical shifts were externally referenced to the carboxylate signal of glycine at 176.4 ppm relative to TMS and were observed at: 39.0, 52.5, 54.9, 64.1, 67.3, 111.6, 114.4, 131.0, 137.0, 144.2, 151.3, 156.4, 158.5, 171.6, 175.5, 177.9 ppm.

### Properties of the Hydrochloride, recorded for the product of Example 1 Solid State Stability of the Hydrochloride

The solid state stability of the drug substance was determined by storing approximately 1.0 g of the material in a glass bottle at a) 40°C / 75% Relative Humidity (RH), open exposure, for 1 month and b) at 50°C, closed, for I month. The material was assayed by HPLC for final content and degradation products in both cases.
a) 40°C / 75% RH: No significant degradation observed (HPLC assay 100% initial).
b) 50°C: No significant degradation observed (HPLC assay 101% initial).

### Solubility of the Hydrochloride

The solubility of the material was determined by adding water in aliquots from 1 to 1000ml to approximately 100mg of drug substance until the powder had dissolved. The visual solubility was confirmed by an HPLC assay of a saturated solution.
Solubility: 25 mg/ml.

### Melting Point of the Hydrochloride

The melting point of the Hydrochloride was determined according to the method described the U.S. Pharmacopoeia, USP 23, 1995, <741> "Melting range or temperature, Procedure for Class Ia", using a Buchi 545 melting point instrument.
Melting Point: 167°C

### Tₒₙₛₑₜ of the Hydrochloride

The Tₒₙₛₑₜ of the drug substance was determined by Differential Scanning Calorimetry using a Perkin-Elmer DSC apparatus.
Product of example 1: Tₒₙₛₑₜ (10°C/minute, open pan): 165°C
Product of example 2: Tₒₙₛₑₜ (10°C/minute, open pan): 163°C

## Claims

1. A compound 5-[4-[2-(N-methyl-N-(2-pyridyl)amino)ethoxy]benzyl]thiazolidine-2,4-dione, hydrochloride salt **characterised in that** it provides:
(i) an infrared spectrum with bands observed at: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617,601, 591, 557, 525, and 505 cm⁻¹; and/or
(ii) a Raman spectrum with bands observed at: 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340, and 300 cm⁻¹ ; and/or
(iii) an X-Ray powder diffraction pattern (XRPD) with characteristic XRPD angles and relative intensities (%) of 5.5 (15.4), 6.7 (14.1), 8.7 (3.3), 11.1 (39.4), 12.0 (9.2), 12.7 (20.3), 13.5, (40.8), 14.7 (40.8), 15.1 (14.5), 15.5 (38.1), 16.3 (30.2), 16.8 (18.5), 17.4 (27.1), 17.7 (34.6), 18.2 (26.7), 18.5 (26), 18.8 (38.8), 19.8 (31), 20.3 (11.7), 21.2 (16.7), 21.7 (26.4), 21.9 (26), 22.5 (14.7), 23.2 (26.2), 23.6 (90.8), 24.2 (100), 25.1, (49.6), 25.5 (68.5), 25.9 (29.3), 26.4 (16.8), 27.3 (25.6), 27.8 (15.4), 28.1 (28.8), 28.8 (21.6), 29.3 (44.1), 29.9 (20.9), 30.5 (17.8), 30.9 (16.8), 31.8 (24), 32.9 (18.7), 33.1 (22), and 34.3 (22.7) °2-theta; and
(iv) a Solid State ¹³C NMR spectrum with chemical shifts observed at: 39.0, 52.5, 54.9, 64.1, 67.3, 111.6, 114.4, 131.0, 137.0, 144.2, 151.3, 156.4, 158.5, 171.6, 175.5, and 177.9 ppm.

2. A compound according to claim 1, **characterised in that** it provides two or more of:
(i) an infrared spectrum with bands observed at: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525, and 505 cm⁻¹;
(ii) a Raman spectrum with bands observed at: 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340, and 300 cm⁻¹ ;
(iii) an X-Ray powder diffraction pattern (XRPD) with characteristic XRPD angles and relative intensities (%) of 5.5 (15.4), 6.7 (14.1), 8.7 (3.3), 11.1 (39.4), 12.0 (9.2), 12.7 (20.3), 13.5, (40.8), 14.7 (40.8), 15.1 (14.5), 15.5 (38.1), 16.3 (30.2), 16.8 (18.5), 17.4 (27.1), 17.7 (34.6), 18.2 (26.7), 18.5 (26), 18.8 (38.8), 19.8 (31), 20.3 (11.7), 21.2 (16.7), 21.7 (26.4), 21.9 (26), 22.5 (14.7), 23.2 (26.2), 23.6 (90.8), 24.2 (100), 25.1, (49.6), 25.5 (68.5), 25.9 (29.3), 26.4 (16.8), 27.3 (25.6), 27.8 (15.4), 28.1 (28.8), 28.8 (21.6), 29.3 (44.1), 29.9 (20.9), 30.5 (17.8), 30.9 (16.8), 31.8 (24), 32.9 (18.7), 33.1 (22), and 34.3 (22.7) °2-theta;
(iv) a Solid State ¹³C NMR spectrum with chemical shifts observed at: 39.0, 52.5, 54.9, 64.1, 67.3, 111.6, 114.4, 131.0, 137.0, 144.2, 151.3, 156.4, 158.5, 171.6, 175.5, and 177.9 ppm; and
(v) a melting point in the range of from 160 to 168°C.

3. A compound according to claim 1 or claim 2, in purified form.

4. A compound according to claim 1 or claim 2, in a solid dosage form.

5. A compound according to claim 1 or claim 2, in a pharmaceutically acceptable form capable of being milled or in a milled form.

6. A compound according to claim 1 or claim 2, in a pharmaceutically acceptable form and having a Hausner ratio of less than or equal to 1.5.

7. A process for preparing the Hydrochloride, according to claim 1, **characterised in that** 5-[4-[2-(N-methyl-N-(2-pyridyl)amino)ethoxy]benzyl]thiazolidine-2,4-dione (Compound I) or salt thereof, preferably dispersed or dissolved in a suitable solvent, is reacted with a source of chloride ion; and thereafter the Hydrochloride is recovered.

8. A pharmaceutical composition comprising the Hydrochloride according to claim 1, and a pharmaceutically acceptable carrier therefor.

9. A Hydrochloride according to claim 1, for use as an active therapeutic substance.

10. A use of the Hydrochloride according to claim 1 for the manufacture of a medicament for the treatment and/or prophylaxis of diabetes mellitus, conditions associated with diabetes mellitus and certain complications thereof.

## Patentansprüche

1. Verbindung 5-[4-[2-(N-Methyl-N-(2-pyridyl)amino)ethoxy]benzyl]thiazolidin-2,4-dion, Hydrochloridsalz, **dadurch gekennzeichnet, daß** sie folgendes liefert:
(i) ein Infrarotspektrum mit Banden, die bei: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525 und 505 cm⁻¹ zu beobachten sind; und/oder
(ii) ein Raman-Spektrum mit Banden, die bei: 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340 und 300 cm⁻¹ zu beobachten sind; und/oder
(iii) ein Röntgenpulverbeugungsmuster (XRPD) mit charakteristischen XRPD-Winkeln und relativen Intensitäten (%) von 5,5 (15,4), 6,7 (14,1), 8,7 (3,3), 11,1 (39,4), 12,0 (9,2), 12,7 (20,3), 13,5 (40,8), 14,7 (40,8), 15,1 (14,5), 15,5 (38,1), 16,3 (30,2), 16,8 (18,5), 17,4 (27,1), 17,7 (34,6), 18,2 (26,7), 18,5 (26), 18,8 (38,8), 19,8 (31), 20,3 (11,7), 21,2 (16,7), 21,7 (26,4), 21,9 (26), 22,5 (14,7), 23,2 (26,2), 23,6 (90,8), 24,2 (100), 25,1 (49,6), 25,5 (68,5), 25,9 (29,3), 26,4 (16,8), 27,3 (25,6), 27,8 (15,4), 28,1 (28,8), 28,8 (21,6), 29,3 (44,1), 29,9 (20,9), 30,5 (17,8), 30,9 (16,8), 31,8 (24), 32,9 (18,7), 33,1 (22) und 34,3 (22,7) °2-theta; und
(iv) ein Festkörper-¹³C-NMR-Spektrum mit chemischen Verschiebungen, die bei: 39,0, 52,5, 54,9, 64,1, 67,3, 111,6, 114,4, 131,0, 137,0,144,2, 151,3, 156,4, 158,5, 171,6, 175,5 und 177,9 ppm zu beobachten sind.

2. Verbindung nach Anspruch 1, **dadurch gekennzeichnet, daß** sie zwei oder mehrere der folgenden liefert:
(i) ein Infrarotspektrum mit Banden, die bei: 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525 und 505 cm⁻¹ zu beobachten sind;
(ii) ein Raman-Spektrum mit Banden, die bei: 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340 und 300 cm⁻¹ zu beobachten sind;
(iii) ein Röntgenpulverbeugungsmuster (XRPD) mit charakteristischen XRPD-Winkeln und relativen Intensitäten (%) von 5,5 (15,4), 6,7 (14,1), 8,7 (3,3), 11,1 (39,4), 12,0 (9,2), 12,7 (20,3), 13,5 (40,8), 14,7 (40,8), 15,1 (14,5), 15,5 (38,1), 16,3 (30,2), 16,8 (18,5), 17,4 (27,1), 17,7 (34,6), 18,2 (26,7), 18,5 (26), 18,8 (38,8), 19,8 (31), 20,3 (11,7), 21,2 (16,7), 21,7 (26,4), 21,9 (26), 22,5 (14,7), 23,2 (26,2), 23,6 (90,8), 24,2 (100), 25,1 (49,6), 25,5 (68,5), 25,9 (29,3), 26,4 (16,8), 27,3 (25,6), 27,8 (15,4), 28,1 (28,8), 28,8 (21,6), 29,3 (44,1), 29,9 (20,9), 30,5 (17,8), 30,9 (16,8), 31,8 (24), 32,9 (18,7), 33,1 (22) und 34,3 (22,7) °2-theta;
(iv) ein Festkörper-¹³C-NMR-Spektrum mit chemischen Verschiebungen, die bei: 39,0, 52,5, 54,9, 64,1, 67,3, 111,6, 114,4, 131,0, 137,0, 144,2, 151,3, 156,4, 158,5, 171,6, 175,5 und 177,9 ppm zu beobachten sind; und
(v) einen Schmelzpunkt im Bereich von 160 bis 168°C.

3. Verbindung nach Anspruch 1 oder Anspruch 2 in gereinigter Form.

4. Verbindung nach Anspruch 1 oder Anspruch 2 in einer festen Dosierungsform.

5. Verbindung nach Anspruch 1 oder Anspruch 2 in einer pharmazeutisch verträglichen Form, die gemahlen werden kann oder in gemahlener Form vorliegt.

6. Verbindung nach Anspruch 1 oder Anspruch 2 in einer pharmazeutisch verträglichen Form und mit einem Hausner-Verhältnis von weniger als oder gleich 1,5.

7. Verfahren zur Herstellung des Hydrochlorids nach Anspruch 1, **dadurch gekennzeichnet, daß** 5-[4-[2-(N-Methyl-N-(2-pyridyl)amino)ethoxy]benzyl]thiazolidin-2,4-dion (Verbindung I) oder dessen Salz, vorzugsweise dispergiert oder gelöst in einem geeigneten Lösungsmittel, mit einer Chloridionenquelle umgesetzt wird und anschließend das Hydrochlorid gewonnen wird.

8. Arzneimittel umfassend das Hydrochlorid nach Anspruch 1 und einen pharmazeutisch verträglichen Träger dafür.

9. Hydrochlorid nach Anspruch 1 zur Verwendung als therapeutischer Wirkstoff.

10. Verwendung des Hydrochlorids nach Anspruch 1 zur Herstellung eines Medikaments zur Behandlung und/oder Prophylaxe von Diabetes mellitus, Zuständen, die mit Diabetes mellitus in Zusammenhang stehen, und bestimmten Komplikationen derselben.

## Revendications

1. Composé consistant en le sel chlorhydrate de 5-[4-[2-(N-méthyl-N-(2-pyridyl)amino)éthoxy]benzyl]thiazolidine-2,4-dione, **caractérisé en ce qu'**il présente :
(i) un spectre infrarouge avec des bandes observées à : 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525 et 505 cm⁻¹ ; et/ou
(ii) un spectre Raman avec des bandes observées à : 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340 et 300 cm⁻¹ ; et/ou
(iii) un diagramme de diffraction des rayons X sur poudre (XRPD) avec des angles et intensités relatives (%) de XRPD caractéristiques de 5,5 (15,4), 6,7 (14,1), 8,7 (3,3), 11,1 (39,4), 12,0 (9,2), 12,7 (20,3), 13,5 (40,8), 14,7 (40,8), 15,1 (14,5), 15,5 (38,1), 16,3 (30,2), 16,8 (18,5), 17,4 (27,1), 17,7 (34,6), 18,2 (26,7), 18,5 (26), 18,8 (38,8), 19,8 (31), 20,3 (11,7), 21,2 (16,7), 21,7 (26,4), 21,9 (26), 22,5 (14,7), 23,2 (26,2), 23,6 (90,8), 24,2 (100), 25,1 (49,6), 25,5 (68,5), 25,9 (29,3), 26,4 (16,8), 27,3 (25,6), 27,8 (15,4), 28,1 (28,8), 28,8 (21,6), 29,3 (44,1), 29,9 (20,9), 30,5 (17,8), 30,9 (16,8), 31,8 (24), 32,9 (18,7), 33,1 (22) et 34,3 (22,7) °2-thêta ; et
(iv) un spectre de ¹³C-RMN à l'état solide avec des déplacements chimiques observés à : 39,0, 52,5, 59,9, 64,1, 67,3, 111,6, 114,4, 131,0, 137,0, 144,2, 151,3, 156,4, 158,5, 171,6, 175,5 et 117,9 ppm.

2. Composé suivant la revendication 1, **caractérisé en ce qu'**il présente deux ou plus de deux des caractéristiques suivantes :
(i) un spectre infrarouge avec des bandes observées à : 2748, 1742, 1697, 1640, 1604, 1543, 1511, 1413, 1351, 1329, 1251, 1232, 1205, 1179, 1159, 1108, 1057, 1028, 996, 973, 925, 902, 817, 764, 737, 710, 662, 617, 601, 591, 557, 525 et 505 cm⁻¹ ;
(ii) un spectre Raman avec des bandes observées à : 3100, 3056, 2931, 1744, 1610, 1586, 1544, 1461, 1440, 1377, 1351, 1319, 1289, 1253, 1232, 1208, 1177, 1095, 1028, 998, 980, 925, 900, 840, 825, 772, 740, 714, 664, 637, 619, 606, 469, 430, 397, 340 et 300 cm⁻¹ ;
(iii) un diagramme de diffraction des rayons X sur poudre (XRPD) avec des angles et intensités relatives (%) de XRPD caractéristiques de 5,5 (15,4), 6,7 (14,1), 8,7 (3,3), 11,1 (39,4), 12,0 (9,2), 12,7 (20,3), 13,5 (40,8), 14,7 (40,8), 15,1 (14,5), 15,5 (38,1), 16,3 (30,2), 16,8 (18,5), 17,4 (27,1), 17,7 (34,6), 18,2 (26,7), 18,5 (26), 18,8 (38,8), 19,8 (31), 20,3 (11,7), 21,2 (16,7), 21,7 (26,4), 21,9 (26), 22,5 (14,7), 23,2 (26,2), 23,6 (90,8), 24,2 (100), 25,1 (49,6), 25,5 (68,5), 25,9 (29,3), 26,4 (16,8), 27,3 (25,6), 27,8 (15,4), 28,1 (28,8), 28,8 (21,6), 29,3 (44,1), 29,9 (20,9), 30,5 (17,8), 30,9 (16,8), 31,8 (24), 32,9 (18,7), 33,1 (22) et 34,3 (22,7) °2-thêta ;
(iv) un spectre de ¹³C-RMN à l'état solide avec des déplacements chimiques observés à : 39,0, 52,5, 54,9, 64,1, 67,3, 111,6, 114,4, 131,0, 137,0, 144,2, 151,3, 156,4, 158,5, 171,6, 175,5 et 117,9 ppm ; et
(v) un point de fusion compris entre 160 et 168°C.

3. Composé suivant la revendication 1 ou la revendication 2, sous forme purifiée ;

4. Composé suivant la revendication 1 ou la revendication 2, sous une forme posologique solide.

5. Composé suivant la revendication 1 ou la revendication 2, sous une forme pharmaceutiquement acceptable pouvant être broyée ou bien sous une forme broyée.

6. Composé suivant la revendication 1 ou la revendication 2, sous une forme pharmaceutiquement acceptable ayant un rapport Hausner inférieur ou égal à 1,5.

7. Procédé pour la préparation du chlorhydrate, suivant la revendication 1, **caractérisé en ce que** de la 5-[4-[2-(N-méthyl-N-(2-pyridyl)amino)éthoxy]benzyl]thiazolidine-2,4-dione (Composé I) ou un de ses sels, de préférence dispersé ou dissous dans un solvant convenable, est amené à réagir avec une source d'ions chlorure, puis le chlorhydrate est recueilli.

8. Composition pharmaceutique comprenant le chlorhydrate suivant la revendication 1 et un support pharmaceutiquement acceptable à cette fin.

9. Chlorhydrate suivant la revendication 1, destiné à être utilisé comme substance thérapeutique active.

10. Utilisation du chlorhydrate suivant la revendication 1 pour la production d'un médicament destiné au traitement et/ou à la prophylaxie du diabète sucré, d'affections associées au diabète sucré et de certaines de leurs complications.
